# EUROPEAN PATENT SPECIFICATION

(11) **EP 1 666 012 B1**
(45) Date of publication and mention of the grant of the patent: **11.04.2012**
(21) Application number: 04772898.5
(22) Date of filing: 02.09.2004
(51) Int. Cl.: A61F 13/49

(54) **PANTS TYPE DISPOSABLE DIAPER AND METHOD OF MANUFACTURING THE DIAPER**
EINWEG-WINDEL VOM HÖSCHENTYP UND VERFAHREN ZUR HERSTELLUNG DER WINDEL
COUCHE CULOTTE A JETER ET SON PROCEDE DE FABRICATION

(30) Priority: 03.09.2003 JP 2003311717
(43) Date of publication of application: 07.06.2006
(73) Proprietor: Livedo Corporation, Shikokuchuo-shi, Ehime 799-0122 (JP)
(72) Inventor: HARUKI, Kaoru,c/o Tokushima Sadamitsu Plant in, cho , Mima-gun, Tokushima 7794104 (JP); HOSHIKAWA, Tadashi, Livedo Corporation, Osaka-shi, Osaka 5410048 (JP)
(74) Representative: Daniels, Jeffrey Nicholas
(86) International application number: PCT/JP2004/013084
(87) International publication number: WO 2005/023160

(56) References cited:
- WO-A-02/30347
- WO-A-2004/026205
- WO-A-2004/064872
- WO-A1-01/95845
- JP-A- 10 298 801
- JP-A- 2002 069 868
- JP-A- 2003 285 890

## Description

### TECHNICAL FIELD

The present invention relates to a pants-type disposable diaper and a process for its production.

### BACKGROUND ART

There have been known so far pants-type disposable diapers (hereinafter referred to as the pants-type diaper(s)), each comprising a diaper main body having a front abdomen portion, a back portion, and leg hole sections which are formed on both sides of a crotch portion between these portions, and further having a waist opening which is formed by joining each of both side edges of the front abdomen portion to corresponding each one of both side edges of the back portion, in which waist elastic members are attached in an expanded state to parts of the front abdomen portion and to parts of the back portion, both of which parts are corresponding to the waist opening, with each of the leg hole sections being provided with a leg-hole elastic member which is attached in an expanded state along the leg holes.

With respect to the pants-type diapers of this kind, since the diaper main body is kept in a shrunk state by the elastic forces of the waist elastic members and the leg-hole elastic members, it has been difficult to recognize identification information including the size and the front or back side of the diaper main body, and the like, when the wearer puts on the diaper. Thus, there have been known pants-type diapers which make it possible to recognize the identification information in wearing the diaper, as disclosed in, for example, Japanese Patent Laid-Open Publication No. 10-298801 and Japanese Patent Laid-Open Publication No. 2000-266.

The pants-type diaper disclosed in Japanese Patent Laid-Open Publication No. 10-298801 is provided with characters attached to the inside of the waist opening, which makes it possible to recognize the front or back side of the diaper, whereas the pants-type diaper disclosed in Japanese Patent Laid-Open Publication No. 2000-266 is provided with patterns printed on the back portion of the diaper main body.

In wearing the pants-type diaper disclosed in the above-identified first patent document, the wearer is allowed to confirm the characters attached to the inside of the waist opening when the waist opening is widened, and can therefore recognize the front or back side of the pants-type diaper. On the other hand, in wearing the pants-type diaper disclosed in the above-identified second patent document, the wearer is allowed to recognize the side bearing the printed patterns as the rear side of the diaper main body by confirming the front or back side of the diaper main body, and can therefore recognize the front or back side of the pants-type diaper.

In the nursing-care facilities and the like, in which pants-type diapers of two or more kinds are daily used, two or more pants-type diapers, which have been taken out of their packaging bags, may often be stacked on one another and stored so as to be promptly used. In the case when the pants-type diapers disclosed in the above-identified first patent document are stored in a stacked state, in order to recognize identification information including the size and the front or back side of each of the diapers, and the like, it requires time-consuming and complicated work, for example, taking out a specific one from the stacked pants-type diapers and then expanding the waist opening of the pants-type diaper. In the case when the pants-type diapers disclosed in the second patent document are stacked on one another and stored, since the patterns printed on the back portion thereof is hidden by other pants-type diapers stacked thereon, it also requires time-consuming and complicated work, for example, taking out a specific one from the stacked pants-type diapers and then confirming the front or back side of this pants-type diaper, in order to recognize the identification information.

The present invention has been completed so as to solve the above-described problems, and an objective of the present invention is to provide a pants-type disposable diaper which enables easy recognition of identification information including the size and the front or back side of each of the diapers, and the like, even when these diapers are placed in a stacked state, and a process for producing such a diaper.

### DISCLOSURE OF THE INVENTION

In order to solve the above-described problems, the present invention provides a pants-type disposable diaper comprising a diaper main body having a front abdomen portion, a back portion, and leg hole sections which are formed on both sides of a crotch portion between these portions, and further having a waist opening which is formed by joining each of both side edges of the front abdomen portion to corresponding each one of both side edges of the back portion, with the crotch portion being provided with an absorbent main body, in-which waist elastic members are attached in an expanded state in a diaper width direction to parts of the front abdomen portion and to parts of the back portion, both of which parts are corresponding to the waist opening, with each of the leg hole sections being provided with a leg-hole elastic member which is attached in an expanded state along the leg hole,
wherein the diaper main body is provided with a display part which displays identification information including the size and the front or back side of the diaper main body, and the like, so as to allow the display part to become recognizable from a side position of each of the diaper main bodies, when two or more diaper main bodies are stacked on one another with the front abdomen portion of one diaper main body being overlapped with the back portion of another diaper main body.

According to the present invention, in the case when two or more pants-type disposable diapers are stacked on one another, since the display part can be confirmed from a side portion of each of the diaper main bodies of the pants-type diapers, the size and the front or back side of each of the diaper main bodies can be recognized in such a state that the respective diaper main bodies are stacked on one another. Moreover, when the display part of the present invention is confirmed in wearing a diaper, the wearer can appropriately wear the pants-type diaper without erroneously recognizing the size and the front or back side of the diaper main body.

The process of the present invention, which is a process for producing a pants-type disposable diaper in which a diaper main body is formed by turning up a sheet constituting a front abdomen portion and a back portion at a position corresponding to a crotch portion, comprises the steps of:
feeding a continuous sheet composed of the two or more sheets, which are joined to each other in parallel to a turn-up line thereof, in a direction parallel to the turn-up line thereof;
opening positions corresponding to leg-hole sections of the continuous sheet; and
intermittently printing identification information including the size and the front or back side of the diaper main body, and the like, on a position which is placed on the outside of the sheet having openings in turning up of the sheet in the vicinity of the turn-up line thereof, by means of an ink-jet system.

According to the process for producing a pants-type disposable diaper of the present invention, since the display parts are intermittently printed on the sheets having openings as leg-hole sections by means of an ink-jet system, it becomes possible to reduce the amount of ink required for printing at positions on the continuous sheet corresponding to the leg-hole sections, resulting in a reduction in the costs of the pants-type diaper, in comparison with the case where display parts are continuously printed along the turn-up line on a continuous sheet without openings as leg-hole sections, followed by opening the leg-hole sections.

### BRIEF DESCRIPTION OF DRAWINGS

FIG. 1 is a perspective view showing a pants-type disposable diaper according to one embodiment of the present invention.
FIG. 2 is a sectional view taken along line II-II of FIG. 1.
FIG. 3 is a perspective view showing a state after the diaper main body of FIG. 1 has been assembled.
FIG. 4 is a plan view showing a producing process of the pants-type disposable diaper of FIG. 1.
FIG. 5 is a schematic side view showing a processing apparatus used for the producing process in FIG. 4.
FIG. 6 is a sectional view taken along line VI-VI of FIG. 1.
FIG. 7 is a front view that shows a diaper main body on which a display part has been formed according to another embodiment.
FIG. 8 is a plan view showing a folding process of a diaper main body when placed into a packaging bag; FIG. 8 (a) shows a state prior to the folding process; FIG. 8 (b) shows a state in which both side edges have been folded; and FIG. 8 (c) shows a state in which a lower edge has been folded.
FIG. 9 is a plan view showing a folding process of a diaper main body when placed into a packaging bag; FIG. 9 (a) shows state in which both side edges have been folded; FIG. 9 (b) shows a state in which a lower edge has been folded; and FIG. 9 (c) shows a state in which the lower edge is further folded.
FIG. 10 is a perspective view showing a display part formed on a diaper main body which has been folded; FIG. 10(a) shows the diaper main body which has been folded as shown in FIG. 8; and FIG. 10 (b) shows the diaper main body which has been folded as shown in FIG. 9.
FIG. 11 is a perspective view showing a state in which diaper main bodies, with display parts being respectively formed thereon, are stacked on one another, according to still another embodiment.

### BEST MODE FOR CARRYING OUT THE INVENTION

A pants-type disposable diaper according to the present invention is a pants-type disposable diaper comprising a diaper main body having a front abdomen portion, a back portion, and leg hole sections which are formed on both sides of a crotch portion between these portions, and further having a waist opening which is formed by joining each of both side edges of the front abdomen portion to corresponding each one of both side edges of the back portion, with the crotch portion being provided with an absorbing main body, in which waist elastic members are attached in an expanded state in a diaper width direction to parts of the front abdomen portion and to parts of the back portion, both of which parts are corresponding to the waist opening, with each of the leg hole sections being provided with a leg-hole elastic member which is attached in an expanded state along each of the leg holes, wherein the diaper main body is provided with a display part which displays identification information the size and the front or back side of the diaper main body, and the like, so as to allow the display part to become recognizable from a side position of each of diaper main bodies, when two or more diaper main bodies are stacked on one another, with the front abdomen portion of one diaper main body being overlapped with the back portion of another diaper main body.

In the above-described pants-type disposable diaper, the diaper main body is formed by turning up a sheet connecting the front abdomen portion and the back portion at a position corresponding to the crotch portion, and the display part may preferably be formed on the outside part of the turned-up sheet.

In the above-described pants-type disposable diaper, the display part may be formed in any of the preferred embodiments: in which it is extended over both the front abdomen portion and the back portion; in which it is extended toward the front abdomen portion; and in which it is extended toward the back portion.

In the above-described pants-type disposable diaper, no elastic members for leg holes may preferably be attached to a part corresponding to a formation position of the display part in a length direction of the diaper main body.

In the above-described pants-type disposable diaper, the display part may preferably be formed on the folded outside part of the diaper main body which has been folded so as to be placed in a packaging bag.

In the above-described pants-type disposable diaper, the display part may preferably be printed on the diaper main body by means of an ink-jet system.

The preferred embodiments will hereinafter be described in detail by reference to the drawings.

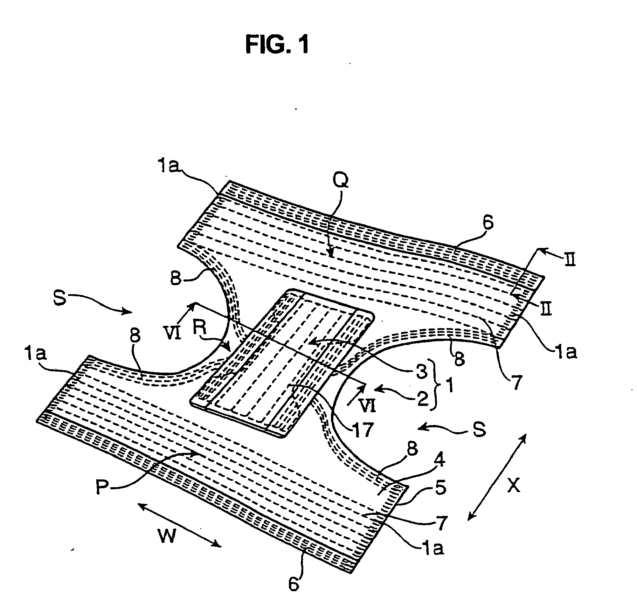

As shown in FIG. 1, a pants-type disposable diaper 1 is basically formed by a diaper main body 2 and an absorbent main body 3.

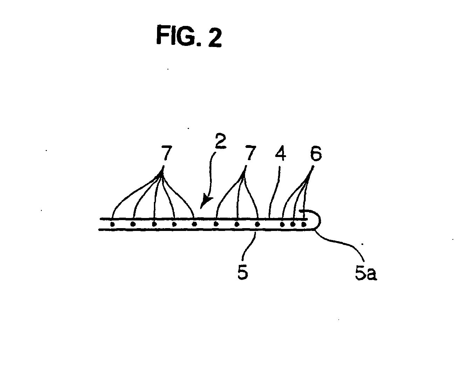

The diaper main body 2, as shown in FIGs. 1 and 2, is provided with an inner sheet (wearer's skin side) 4 in which leg-hole sections S are formed on both sides of a crotch portion R between a front abdomen portion P and a back portion Q, and an outer sheet (outer face side) 5 (both of which are corresponding to a sheet). On waist sections of the front abdomen portion P and the back portion Q between the inner sheet 4 and the outer sheet 5, two or more waist elastic members 6 are attached and placed in an extended state in a diaper width direction W with narrow intervals in a diaper length (front-to-rear) direction X, while on body sections, two or more body-fitting elastic members 7 are attached and placed in an extended state in a diaper width direction W with wide intervals in a diaper length direction X.

Moreover, on each of the leg-hole sections S, two or more leg-hole elastic members 8 are attached and placed in an extended state along the leg hole between the inner sheet 4 and the outer sheet 5. These leg-hole elastic members 8 are attached and placed at positions except for an approximately center position (a part corresponding to the formation position of a display part 20, which will be described later) of the crotch portion R in the diaper length direction X.

The inner sheet 4 and the outer sheet 5 are bonded to each other by a hot-melt adhesive or the like, with the respective elastic members 6 to 8 being sandwiched therebetween. In addition, a margin portion 5a elongated in the diaper length direction X on each of both ends of the external sheet 5 is bent over in a manner so as to wrap the corresponding each one of both ends of the inner sheet 4, and is bonded to the corresponding each one of both ends of the inner sheet 4 (see Fig. 2).

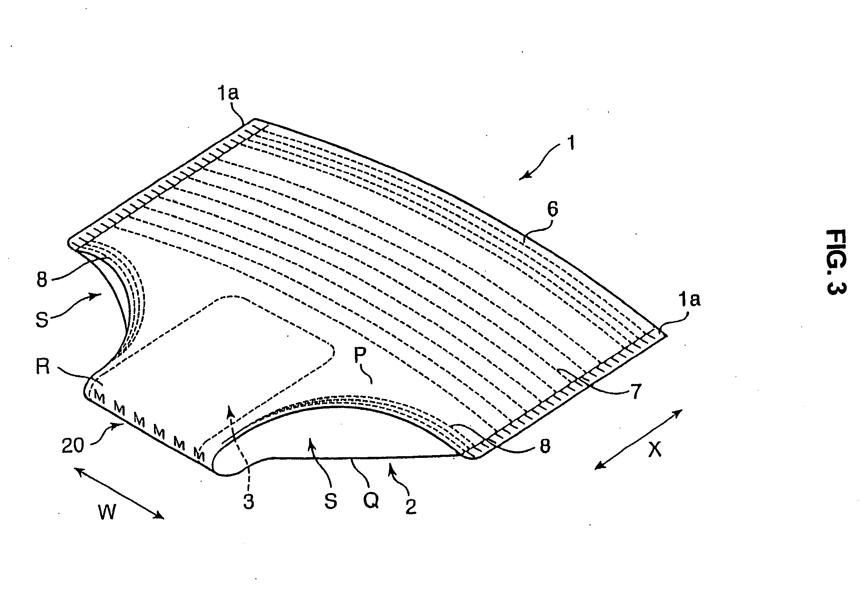

The diaper main body 2, as will be described later in detail, is turned up in a manner so as to overlap the front abdomen portion P with the back portion Q with the inner sheet 4 located inside thereof so that each of side edges 1a of the front abdomen portion P is joined to corresponding each one of side edges 1a of the back portion Q. At this time, the diaper main body 2 is turned up at an approximately center position of the crotch portion R in the diaper length direction X, and a display part 20 is formed on a part on the outside of the turning-up position, that is, on the outer sheet 5, as shown in Fig. 3.

On the display part 20, in the present embodiment, characters "M" which indicate the size of the diaper main body 2 (size M: one example of identification information) are aligned in one row along the diaper width direction W on the display part 20, and these characters "M" are printed in a direction so as to be read in the case when the diaper main bodies 2 are placed with the front abdomen portion P facing up.

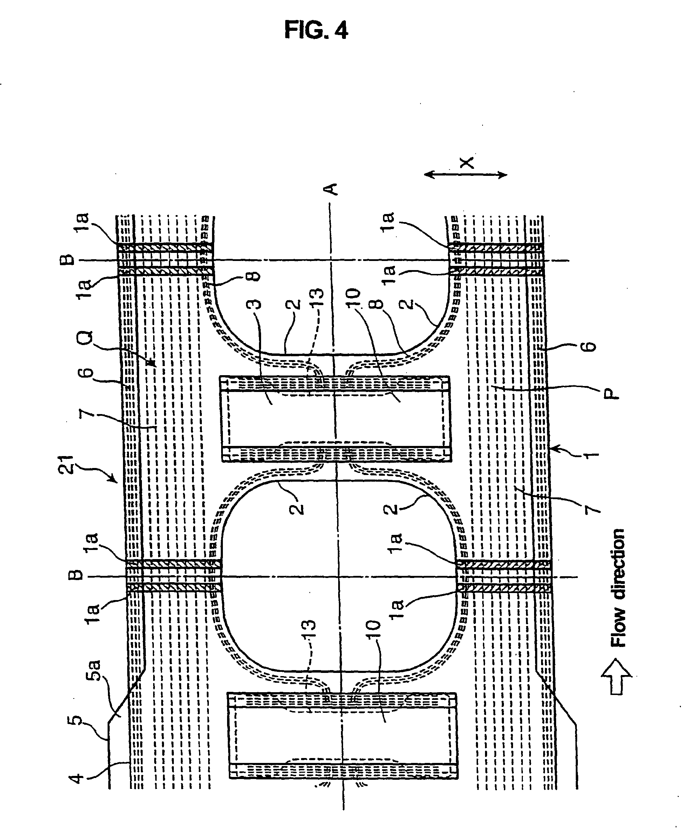

In the case when the above-described diaper main bodies 2 are produced in a laterally flowing state in the diaper width direction W, as shown in Fig. 4, a continuous sheet 21 of the diaper main body 2 on which the display part 20 has been formed is turned up along a turn-up line A (that is, the center line of the crotch portion R) so that waist sections of the front abdomen portion P and the back portion Q are position-adjusted; thus, after the front abdomen portion P and the back portion Q have been joined to each other at their side edges 1a by thermal fusing or the like, the sheet is then cut into individual diaper main bodies 2 along the cut line B. In this manner, a diaper main body 2 with waist openings is assembled by joining both corresponding edges 1a, 1a to each other.

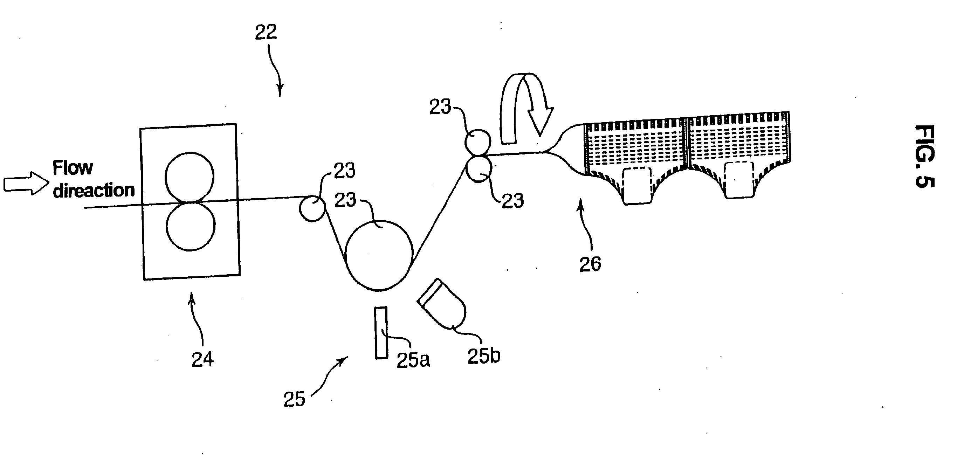

As described above, in the case when the continuous sheet 21 in which each of the inner sheets 4 and each of the outer sheets 5 are connected to one another in parallel to the turn-up line A is processed through the laterally flowing process, more specifically, a processing apparatus 22, as shown in Fig. 5, is used. This processing apparatus 22 is provided with two or more transporting rollers 23 used for transporting the continuous sheet 21 along a transport path in parallel to the turn-up line A, a leg cutter 24 placed on the upstream side of the transport path, a printing device 25 placed on the downstream side of the leg cutter 24 and a turn-up unit 26 placed on the downstream side of this printing device 25.

The leg cutter 24 is designed so as to open positions corresponding to the leg-holes S in the continuous sheet 21 which has been transported.

The printing device 25 is provided with a nozzle 25a which discharges ink in accordance with a predetermined printing pattern, and a drying device 25b which dries ink on the downstream side of this nozzle 25a, and intermittently prints display parts 20 on the outer sheet 5 at positions corresponding to the turn-up line A of the continuous sheet 21 in which the leg-hole sections S have been formed by the leg cutter 24.

The turn-up unit 26, which is omitted from this figure, is provided with a mechanism which turns up the continuous sheet 21 along the turn-up line A.

The continuous sheet 21 processed by the processing device 22 is then joined to one another at both side edges 1a as described above, and then cut into individual diaper main bodies 2 along the cut-line B.

As the inner sheet 4, there can be used a water-repellant non-woven fabric material (span-bond non-woven fabric, melt-blow non-woven fabric, SMS non-woven fabric, or the like). Preferably used is a water-repellant non-woven fabric material with a METSUKE (basis weight per unit area) of 10 to 25 g/m². Moreover, only the inner face may be subjected to hydrophilic treatment [coated with a surfactant, or a layer of hydrophilic fibers (rayon, cotton, or the like) is formed thereon], and this treatment provides better feelings when brought into contact with the skin, and improves the sweat absorbing effects.

As the outer sheet 5, there can be used a water-repellant non-woven fabric material (span-bond non-woven fabric, melt-blow non-woven fabric, SMS non-woven fabric, or the like). Preferably used is a water-repellant non-woven fabric material with a METSUKE (basis weight per unit area) of 10 to 25 g/m².

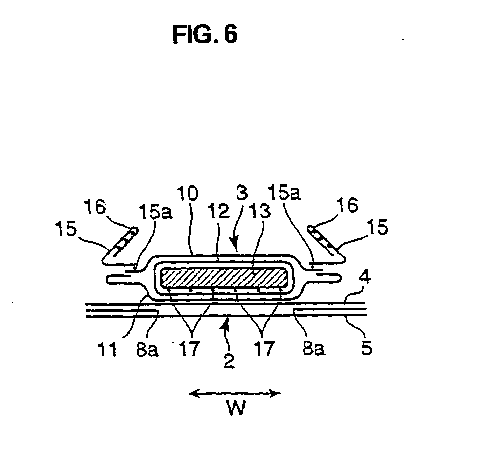

The above-described absorbent main body 3, as shown in Figs. 4 and 6, is formed by sandwiching an absorbent core 13 having an approximately sandglass shape wrapped with a coating sheet 12, between a top sheet 10 and a back sheet 11, and is extended and placed in the diaper length direction X.

To both sides of the inner face (top face) of the top sheet 10 of the above-described absorbent main body 3, base ends 15a of rising flaps 15 which are extended and placed in the diaper length direction X are respectively bonded, and raised upward so that their upper ends are turned up inwardly, with flap elastic members 16 being attached and placed within the turned-up portion in an extended state in the diaper length direction X, so that the rising flaps 15 are allowed to rise. The inner faces of the front and rear ends of the rising flap 15 are bonded to the top sheet 10 in a fallen state.

Between the coating sheet 12 and the absorbent core 13 of the above-described absorbent main body 3, two or more crotch elastic members 17 are attached and placed on the absorbent core 13 in an extended state in the length direction X with predetermined intervals over the entire width in the diaper width direction W.

The absorbent core 13 of the above-described absorbent main body 3 is bonded to the top sheet 10 and the back sheet 11 over the almost entire face thereof using a hot-melt adhesive or the like, and the back sheet 11 is bonded to the inner sheet 4 of the crotch portion R of the above-described diaper main body 2 over the almost entire face using a hot-melt adhesive or the like.

The absorbent core 13 is formed through the following processes: a material prepared by mixing particle-shaped polymer absorbent material into a hydrophilic fiber aggregate layer made of pulverized pulp fibers, cellulose fibers, or the like, is wrapped with a coating sheet 12 including a paper sheet such as tissue paper or a liquid permeable non-woven fabric sheet, and this wrapped material is formed into a predetermined shape (a rectangular shape, a sandglass shape, a gourd shape, or the like). This wrapped material may be prepared by shaping a sheet-shaped body (for example, air-laid absorbent body) or the like, which is obtained by forming the above-described fibers and polymer absorbent body into a sheet shape, into a predetermined form. Moreover, in order to maintain the shape even when the wearer walks or changes the sides while sleeping, a shape-retaining means (application of a hot-melt adhesive, or mixing of synthetic fibers) may be placed in the hydrophilic fiber aggregated layer or sheet.

The top sheet 10 may preferably be made of a liquid permeable non-woven fabric material. For example, there can be used a non-woven fabric material using hydrophilic fibers (cellulose, rayon, cotton, or the like), or a non-woven fabric material in which the surfaces of hydrophobic fibers (polypropylene, polyester, polyamide, nylon, or the like) are treated by a surfactant to be made liquid permeable. Preferably used are non-woven fabric materials (point-bond non-woven fabric, air through non-woven fabric, and the like) in which one of the surfaces of hydrophobic fibers having a METSUKE (basis weight per unit area) of 10 to 25 g/m² is treated by a surfactant to be made liquid permeable.

As the back sheet 11, there can be used a water repellent non-woven fabric material (span-bond non-woven fabric, melt-blow non-woven fabric, SMS non-woven fabric, or the like), a plastic film (which may be either gas-permeable or gas-impermeable, and may desirably be a gas-permeable plastic film as a preferred embodiment), or their composite materials. Preferably used is a gas-permeable polyethylene film having a METSUKE (basis weight per unit area) of 15 to 40 g/m².

As the rising flap 15, there can be used a water repellent non-woven fabric material (span-bond non-woven fabric, melt-blow non-woven fabric, SMS non-woven fabric, or the like). Preferably used is a water repellent non-woven fabric material with a METSUKE (basis weight per unit area) of 10 to 25 g/m².

As the coating sheet 12, there can be used a paper sheet such as tissue paper or a liquid-permeable non-woven fabric sheet.

As the respective elastic members 6 to 8, 16, and 17, there can be used elastic expansion materials (polyurethane yarns, polyurethane films, natural rubbers, or the like), which are generally used as disposable diapers, and each of these members is attached and placed between the respective sheet materials in an extended state using an appropriate attaching means such as a hot-melt adhesive, thermal joining, or ultrasonic joining. Preferably used is a rubber-type hot-melt adhesive.

As the waist elastic member 6, a polyurethane yarn having a size of 700 to 2,000 dtex may preferably be attached and formed in an extended state with a magnification of 2.0 to 5.0 times.

As the body fit elastic member 7, a polyurethane yarn having a size of 300 to 1, 000 dtex may preferably be attached and formed in an extended state with a magnification of 1.1 to 5.0 times.

As the leg-hole elastic member 8, a polyurethane yarn having a size of 700 to 2,000 dtex may preferably be attached and formed in an extended state with a magnification of 1.1 to 3.0 times.

As the flap elastic member 16, a polyurethane yarn having a size of 300 to 2,000 dtex may preferably be attached and formed in an extended state with a magnification of 1.1 to 4.0 times.

As the crotch elastic member 17, a polyurethane yarn having a size of 300 to 2,000 dtex may preferably be attached and formed in an extended state with a magnification of 1.1 to 4.0 times.

The joining process of each of the sheet materials may appropriately be carried out using a method such as a hot-melt adhesive, thermal joining, or ultrasonic joining. The hot-melt adhesive may appropriately be selected from hot-melt adhesives such as those of the rubber type, the polyolefin type, the vinyl acetate type, or the like, and may be applied using a method such as direct coater application, indirect spiral coating, melt-blow (curtain-spraying) coating, and bead coating. The side edges 1a may preferably be formed by thermal joining

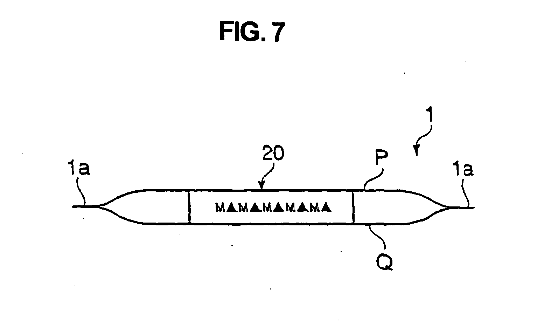

In the above-described embodiment, the size is displayed as identification information for the display part 20; however, the present invention is not limited to this arrangement, but there may be displayed, for example, a pattern such as used for indicating the front or back side of the diaper main body 2, as shown in Fig. 7 (in the present embodiment, "▲" mark indicating the front abdomen P side is used for the display part 20) . In the present embodiment, the characters "M" indicating the size and the marks "▲" indicating the side are alternately arranged along the diaper width direction.

Moreover, in the above-described embodiment, the character on the display part 20 is printed in such a direction as to be read when the diaper main body 2 is placed with the front abdomen portion P facing up; however, the direction of the character on the display part 20 is not limited to this arrangement, but it may be printed, for example, in such a manner as to be read when the diaper main body 2 is placed with the back portion Q facing up.

Further, in the above-described embodiment, the leg-hole elastic member 8 is not attached and placed on a part corresponding to the formation position of the display part 20 in the diaper length direction X; however, the present invention is not limited to this arrangement. For example, the leg-hole elastic member 8 may be attached and placed along the entire circumference of the leg-hole portion 20, and the middle portion of the leg-hole elastic member 8 corresponding to the display part 20 in the diaper length direction X may be cut so that the elastic force may be reduced.

Moreover, in the above described respective embodiments, the display part 20 is formed on the outer sheet 5 at a position corresponding to the turn-up line A of the crotch portion R; however, the present invention is not limited to this arrangement, but the display part 20 may be formed, for example, on the folded outside part of the diaper main body 2 which has been folded so as to be placed into a packaging bag.

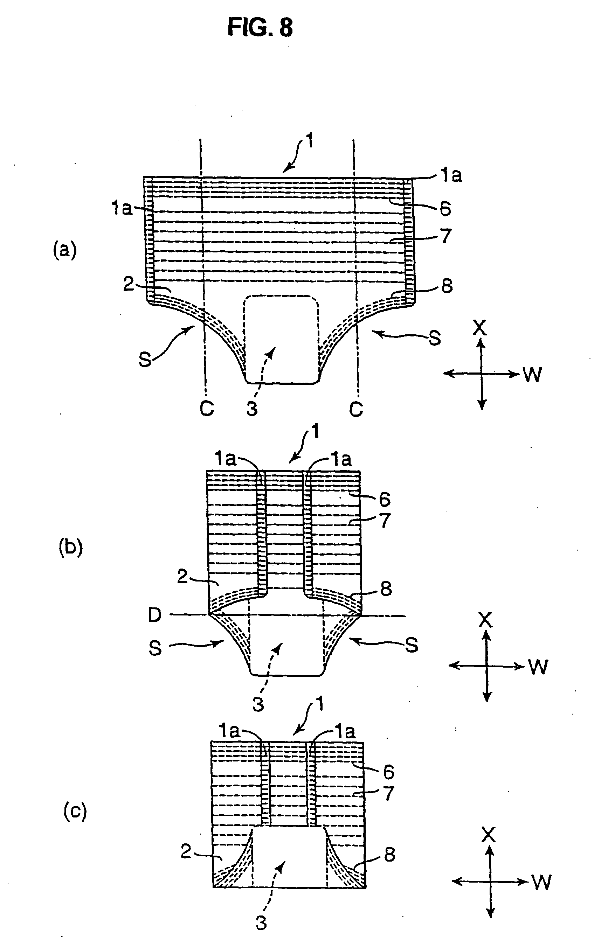

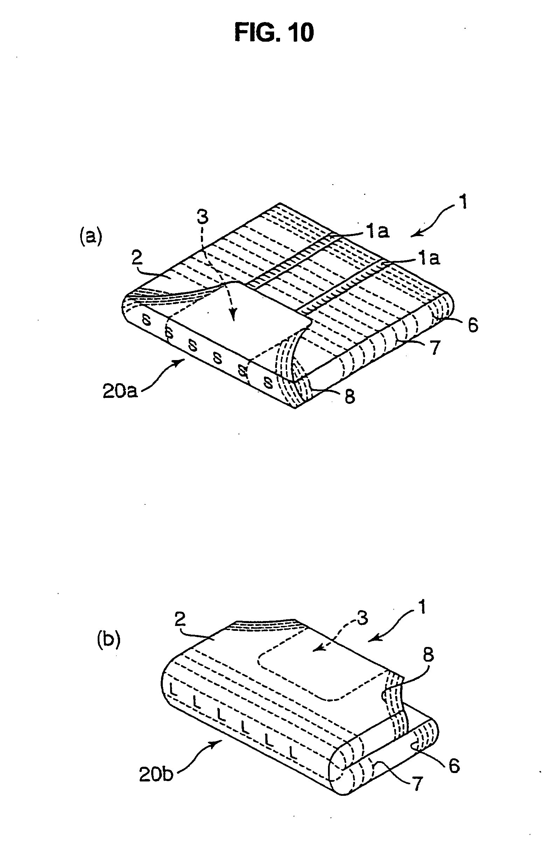

More specifically, in order to place the pants-type diaper 1 into a packaging bag, as shown in Fig. 8 (a), both side edges 1a of the diaper main body 2 are folded inward along a pair of turn-up lines C which extend along the diaper length direction X on both sides of the absorbent main body 3 and, as shown in Fig. 8 (b), the lower end of the diaper main body 2 may be folded along a turn-up line D in a manner so as to cover the folded both side edges 1a. Thus, the diaper main body 2 is folded into an approximately square shape when seen in a plan view, as shown in Fig. 8(c). With respect to the diaper main body 2 folded in this manner, a display part 20a (in the present embodiment, size "S" is shown) may be formed on the outside part which has been turned up along a turn-up line D, as shown in Fig. 10 (a) . In such a folded state, in place of the display part 20a, or in addition thereto, a display part may be formed on the outside part which has been turned up along a turn-up line C.

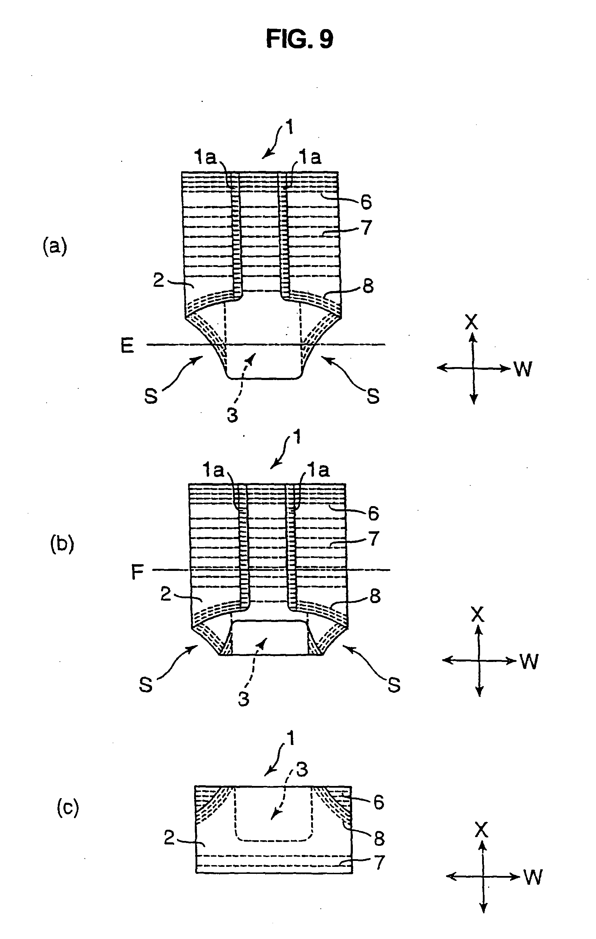

Moreover, in the case when the pants-type diaper 1 is placed into a packaging bag, after both side edges 1a have been folded along the two turn-up lines C, as shown in Fig. 9 (a), the diaper main body 2 may be folded along a turn-up line E extending along the diaper width direction W at a position lower than the turn-up line D, and in this state, the diaper main body 2 may be folded into upper and lower half portions along a turn-up line F shown in Fig. 9 (b) in a manner so as to make the positions of the lower end (turn-up line E) and the upper end (waist opening) coincident with each other; thus, as shown in Fig. 9(c), the diaper main body 2 is folded into an approximately rectangular shape when seen in a plan view, as shown in Fig. 9 (c) . With respect to the diaper main body 2 folded in this manner, as shown in Fig. 10 (b), a display part 20b (in the present embodiment, size "L" is shown) may be formed on the outside part which has been turned up along a turn-up line F. In such a folded state, in place of the display part 20b, or in addition thereto, a display part may be formed on the outside part which has been turned up along turn-up lines C and E.

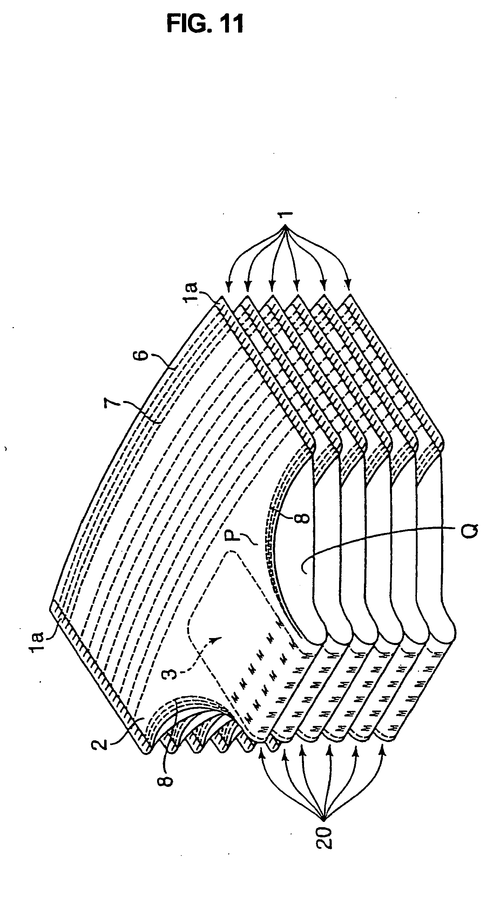

Further, in the above-described respective embodiments, each of the display parts 20, 20a, and 20b is formed only on the outside part of the turned-up sheet; however, there present invention is not limited to this arrangement, but for example, as shown in Fig. 11, the display part 20 may be extended to the front abdomen portion P of the diaper main body 2, that is, the display information of the display part 20 may be displayed also on the front abdomen portion P. The extended portion of the display part 20 is not limited to the front abdomen portion P, and the display part 20 may be formed on the back portion Q, or the display part 20 may be formed on both the front abdomen portion P and the back portion Q.

As described above, according to the pants-type disposable diaper 1, when two or more diaper main bodies 2 are stacked on one another, each of these display parts 20, 20a, and 20b can be recognized from a side position of each of the diaper main bodies 2 so that the size and the front or back side of the diaper main body 2 can be recognized in the stacked state of the diaper main bodies 2. Moreover, by confirming each of the display parts 20, 20a, and 20b in wearing a pants-type diaper, the wearer is allowed to wear a proper pants-type diaper 1, without erroneously recognizing the size and the front or back side of the diaper main body 2.

In the case when the display part 20 is designed so as to be formed on the outside part of the outer sheet 5 turned up along the crotch portion R, it becomes possible to recognize the size and the front or back side of each of the diaper main bodies 2 which are stacked with the crotch portion R facing sideward. Moreover, since the display part 20 is placed the outside part of the folded crotch portion R, that is, since the display part 20 is placed between the thighs of the wearer, the display part 20 is made less conspicuous comparatively when the diaper is used.

With the arrangement that the display part 20 is extended over both the front abdomen portion P and the back portion Q, for example, when two or more diaper main bodies 2 are stacked with respect to each of the sizes (see Fig. 11), by confirming the diaper main body 2 placed on the top of the stack from above, the size of the stack of the diaper main bodies 2 can be recognized.

In the case when the display part 20 is designed so as to be extended toward the front abdomen portion P, in wearing a pants-type diaper, the extended side of the display part 20 can be recognized as the front side of the diaper main body 2.

In the case when the display part 20 is designed so as to be extended toward the back portion Q, in wearing a pants-type diaper, the extended side of the display part 20 can be recognized as the back side of the diaper main body 2.

According to the arrangement in which the leg-hole elastic members 8 are not placed at a position having the display part 20 between the two leg-holes portions S, since this makes it possible to prevent identification information displayed on the display part 20 from shrinking due to an elastic force of the leg-hole elastic members 8, the display part 20 formed on the crotch portion R can more clearly be displayed.

According to the arrangement in which the display parts 20a and 20b are formed on the folded outside parts of the diaper main body 2 which has been folded so as to be placed in a packaging bag, even when the diaper main bodies 2 placed in packaging bags are taken out and stacked as they are, the identification information of the diaper main bodies 2 can be recognized.

By printing the display parts 20, 20a, and 20b by means of an ink-jet system, the display parts 20, 20a, and 20b can be formed by a comparatively easy method, and at the same time, the contents of identification information can comparatively easily be changed.

Moreover, for producing pants-type disposable diapers 1, a continuous sheet 21 composed of two or more inner sheets 4 and two or more outer sheets 5, respectively, forming the front abdomen portions P and the back portions P, which inner sheets and outer sheets are joined to each other in parallel to a turn-up line A, is transported in a direction parallel to the turn-up line A, and after opening positions corresponding to leg-hole sections S of the continuous sheet 21, a display part 20 indicating identification information of the diaper main body 2 is intermittently printed on the outer sheet 5 in the vicinity of the turn-up line A in the sheet with openings, by means of an ink-jet system. For this reason, in comparison with the arrangement in which display parts 20 are continuously printed along the turn-up line A of a continuous sheet without openings for the leg-hole sections S, and then leg-hole sections S are made, the amount of ink required for printing the positions of the continuous sheet 21 corresponding to the leg-hole sections S can be reduced, thereby making it possible to reduce the costs of the ants-type disposable diaper 1.

In the above-described embodiment, the display parts 20, 20a, and 20b are employed for the pants-type disposable diaper 1 in which the absorbent main body 3 is bonded to the inner sheet 4; however, in place of this arrangement, the display parts 20, 20a, and 20b may be applied to a pants-type disposable diaper in which the absorbent main body 3 is disposed between the inner sheet 4 and the outer sheet 5.

### INDUSTRIAL APPLICABILITY

The pants-type disposable diaper of the present invention allows its display part that displays identification information including the size and the front or back side of the diaper, and the like, to become easily recognizable from a side position of each of the diaper main bodies, even when two or more diapers are stacked on one another and stored. Therefore, this arrangement allows employees in the nursing-care facilities or the like where pants-type diapers of two or more kinds are daily used to promptly recognize the identification information, even when two or more pants-type diapers, which have been taken out of packaging bags, stacked on one another, and stored, and consequently to readily take out a pants-type diaper having a required size.

## Claims

1. A pants-type disposable diaper comprising a diaper main body having a front abdomen portion, a back portion, and leg hole sections which are formed on both sides of a crotch portion between these portions, and further having a waist opening which is formed by joining each of both side edges of the front abdomen portion to corresponding each one of both side edges of the back portion, with the crotch portion being provided with an absorbent main body, in which waist elastic members are attached in an expanded state in a diaper width direction to parts of the front abdomen portion and to parts of the back portion, both of which parts are corresponding to the waist opening, with each of the leg hole sections being provided with a leg-hole elastic member which is attached in an expanded state along each of the leg holes,
wherein the diaper main body is provided with a display part which displays identification information including a size and a front or back side of the diaper main body, and the like, so as to allow the display part to become recognizable from a side position of each of diaper main bodies, when two or more diaper main bodies are stacked on one another, with the front abdomen portion of one diaper main body being overlapped with the back portion of another diaper main body.

2. The pants-type disposable diaper according to claim 1, wherein the diaper main body is formed by turning up a sheet connecting the front abdomen portion to the back portion at a position corresponding to the crotch portion, and the display part is formed on an outside part of the turned-up sheet.

3. The pants-type disposable diaper according to claim 2, wherein the display part is extended and formed over both the front abdomen portion and the back portion.

4. The pants-type disposable diaper according to claim 2, wherein the display part is extended and formed toward the front abdomen portion.

5. The pants-type disposable diaper according to claim 2, wherein the display part is extended and formed toward the back portion.

6. The pants-type disposable diaper according to any of claims 2 to 5, wherein no elastic members for leg holes are attached to a part corresponding to a formation position of the display part in a length direction of the diaper main body.

7. The pants-type disposable diaper according to claim 1, wherein the display part is formed on the folded outside part of the diaper main body which has been folded so as to be placed into a packaging bag.

8. The pants-type disposable diaper according to any of claims 1 to 7, wherein the display part is printed on the diaper main body by means of an ink-jet system.

9. A process of producing a pants-type disposal diaper in which a diaper main body is formed by turning up a sheet constituting a front abdomen portion and a back portion at a position corresponding to a crotch portion, comprising steps of:
feeding a continuous sheet composed of the two or more sheets, which are joined to each other in parallel to a turn-up line thereof, in a direction parallel to the turn-up line thereof;
opening positions corresponding to leg-hole sections of the continuous sheet; and
intermittently printing identification information including a size and a front or back side of the diaper main body, and the like, on a position which is placed on an outside of the sheet having openings in turning up of the sheet in a vicinity of the turn-up line thereof, by means of an ink-jet system.

## Patentansprüche

1. Wegwerfwindel vom Höschentyp, umfassend einen Windelhauptkörper, der einen vorderen Bauchabschnitt, einen Rückenabschnitt und Beinöffnungsbereiche aufweist, die an beiden Seiten eines Schrittabschnitts zwischen diesen Abschnitten gebildet sind, und ferner eine Taillenöffnung aufweist, die gebildet wird, indem jede der beiden Seitenkanten des vorderen Bauchabschnitts mit der jeweils entsprechenden der beiden Seitenkanten des Rückenabschnitts verbunden ist, wobei der Schrittabschnitt mit einem absorbierenden Hauptkörper versehen ist, bei dem elastische Taillenteile in gedehntem Zustand in Breitenrichtung der Windel an Teilen des vorderen Bauchabschnitts und an Teilen des Rückenabschnitts befestigt sind, wobei beide der Teile der Taillenöffnung entsprechen, wobei jeder der Beinöffnungsbereiche mit einem elastischen Beinöffnungsteil versehen ist, der in gedehntem Zustand entlang jeder der Beinöffnungen befestigt ist,
wobei der Windelhauptkörper mit einem Anzeigeteil versehen ist, der Identifikationsinformationen, einschließlich Größe und Vorder- oder Rückseite des Windelhauptkörpers, und dergleichen zeigt, so dass der Anzeigeteil von einer Seitenposition eines jeden der Windelhauptkörper erkennbar ist, wenn zwei oder mehr Windelhauptkörper übereinander gestapelt sind, wobei der vordere Bauchabschnitt eines Windelhauptkörpers von dem Rückenabschnitt eines anderen Windelhauptkörpers überlappt wird.

2. Wegwerfwindel vom Höschentyp nach Anspruch 1, wobei der Windelhauptkörper durch Umschlagen einer Schicht gebildet wird, die den vorderen Bauchabschnitt mit dem Rückenabschnitt verbindet, an einer Position, die dem Schrittabschnitt entspricht, und der Anzeigeteil an einem Außenseitenteil der umgeschlagenen Schicht gebildet wird.

3. Wegwerfwindel vom Höschentyp nach Anspruch 2, wobei sich der Anzeigeteil sowohl über den vorderen Bauchabschnitt als auch den Rückenabschnitt erstreckt und darüber gebildet wird.

4. Wegwerfwindel vom Höschentyp nach Anspruch 2, wobei sich der Anzeigeteil in Richtung des vorderen Bauchabschnitts erstreckt und gebildet wird.

5. Wegwerfwindel vom Höschentyp nach Anspruch 2, wobei sich der Anzeigeteil in Richtung des Rückenabschnitts erstreckt und gebildet wird.

6. Wegwerfwindel vom Höschentyp nach einem der Ansprüche 2 bis 5, wobei keine elastischen Teile für Beinöffnungen an einem Teil befestigt sind, der einer Bildungsposition des Anzeigeteils in Längenrichtung des Windelhauptkörpers entspricht.

7. Wegwerfwindel vom Höschentyp nach Anspruch 1, wobei der Anzeigeteil an dem gefalteten Außenseitenteil des Windelhauptkörpers gebildet ist, der so gefaltet worden ist, dass er in einem Verpackungsbeutel platziert werden kann.

8. Wegwerfwindel vom Höschentyp nach einem der Ansprüche 1 bis 7, wobei der Anzeigeteil auf den Windelhauptkörper mittels eines Tintenstrahlsystems gedruckt ist.

9. Verfahren zur Herstellung eines Wegwerfwindel vom Höschentyp, wobei ein Windelhauptkörper durch Umschlagen einer Schicht gebildet wird, die einen vorderen Bauchabschnitt und einen Rückenabschnitt bildet, an einer Position, die einem Schrittabschnitt entspricht, umfassend die Schritte:
Zuführen einer fortlaufenden Schicht, bestehend aus zwei oder mehr Schichten, die miteinander parallel zu deren Umschlaglinie verbunden sind, in einer Richtung parallel zu deren Umschlaglinie;
Öffnen von Positionen, die den Beinöffnungsbereichen der fortlaufenden Schicht entsprechen; und
periodisches Drucken von Identifikationsinformationen, einschließlich Größe und Vorder- oder Rückseite des Windelhauptkörpers, und dergleichen auf eine Position, die sich an einer Außenseite der Schicht befindet, die Öffnungen im Umschlag der Schicht in der Nähe von deren Umschlaglinie aufweist, mittels eines Tintenstrahlsystems.

## Revendications

1. Couche jetable de type culotte comprenant un corps principal de couche présentant une portion abdominale avant, une portion arrière, et des sections de trous pour les jambes qui sont formées sur les deux côtés d'une portion d'entrejambe entre ces portions, et présentant en outre une ouverture de taille qui est formée en assemblant chacun des deux bords latéraux de la portion abdominale avant à chacun des deux bords latéraux correspondants de la portion arrière, la portion d'entrejambe étant pourvue d'un corps principal absorbant, où des éléments élastiques de taille sont fixés, dans un état étendu dans une direction de largeur de la couche, à des parties de la portion abdominale avant et à des parties de la portion arrière, ces deux parties correspondent à l'ouverture de taille, chacune des sections de trous pour les jambes étant pourvue d'un élément élastique de trou pour la jambe qui est fixé dans un état étendu le long de chacun des trous pour les jambes,
où le corps principal de couche est pourvu d'une partie d'affichage qui affiche des informations d'identification comportant une taille et un côté avant ou arrière du corps principal de couche, et autres analogues, afin de permettre la reconnaissance de la partie d'affichage à partir d'une position latérale de chacun des corps principaux de couche, lorsque deux corps principaux ou plus de couche sont empilés les uns sur les autres, avec la portion abdominale avant d'un corps principal de couche recouverte par la portion arrière d'un autre corps principal de couche.

2. Couche jetable de type culotte selon la revendication 1, dans laquelle le corps principal de couche est formé en pliant une feuille reliant la portion abdominale avant à la portion arrière a une position correspondant à la portion d'entrejambe, et la partie d'affichage est formée sur une partie extérieure de la feuille pliée.

3. Couche jetable de type culotte selon la revendication 2, dans laquelle la partie d'affichage est formée à la fois sur la portion abdominale avant et la portion arrière et s'étend sur celles-ci.

4. Couche jetable de type culotte selon la revendication 2, dans laquelle la partie d'affichage est formée et s'étend vers la portion abdominale avant.

5. Couche jetable de type culotte selon la revendication 2, dans laquelle la partie d'affichage est formée et s'étend vers la portion arrière.

6. Couche jetable de type culotte selon l'une des revendications 2 à 5, dans laquelle aucun des éléments élastiques destinés aux trous pour les jambes n'est fixé à une partie correspondant à une position de formation de la partie d'affichage dans une direction de longueur du corps principal de couche.

7. Couche jetable de type culotte selon la revendication 1, dans laquelle la partie d'affichage est formée sur la partie extérieure pliée du corps principal de couche qui a été pliée de manière à être placée dans un sac d'emballage.

8. Couche jetable de type culotte selon l'une des revendications 1 à 7, dans laquelle la partie d'affichage est imprimée sur le corps principal de couche en utilisant un système à jet d'encre.

9. Procédé de production d'une couche jetable de type culotte, où un corps principal de couche est formé en pliant une feuille constituant une portion abdominale avant et une portion arrière à une position correspondant à une portion d'entrejambe, comprenant les étapes qui consistent :
à fournir une feuille continue composée de deux feuilles ou plus, qui sont reliées les unes aux autres parallèlement à sa ligne de pliage, dans une direction parallèle à sa ligne de pliage ;
à ouvrir des positions correspondant à des sections de trous pour les jambes de la feuille continue ; et
à imprimer de manière intermittente des informations d'identification comportant la taille et un côté avant ou arrière du corps principal de couche, et analogues, sur une position qui est placée sur l'extérieur de la feuille ayant des ouvertures à l'étape de pliage de la feuille à proximité de sa ligne de pliage, en utilisant un système à jet d'encre.
